# EUROPEAN PATENT SPECIFICATION

(11) **EP 4 316 558 B1**
(45) Date of publication and mention of the grant of the patent: **09.07.2025**
(21) Application number: 22188533.8
(22) Date of filing: 03.08.2022
(51) Int. Cl.: A61M 16/06, A61M 15/00

(54) **INHALATION MASK**
INHALATIONSMASKE
MASQUE D'INHALATION

(43) Date of publication of application: 07.02.2024
(73) Proprietor: PARI GmbH Spezialisten für effektive Inhalation, 82319 Starnberg (DE)
(72) Inventor: Peter, Christian, 82239 Alling (DE); Winzen, Dr. Andrea, 82166 Gräfelfing (DE)
(74) Representative: Hoffmann Eitle

(56) References cited:
- EP-A1- 3 082 924
- EP-A1- 3 347 076
- WO-A1-2011/121463
- WO-A1-2013/186650
- WO-A1-2017/158471
- WO-A1-2017/199215
- WO-A1-2018/104253
- WO-A1-2020/073840
- WO-A1-2021/019312
- WO-A2-2005/041820
- US-A1- 2008 178 886
- US-A1- 2010 294 281
- US-A1- 2017 128 689

## Description

The present invention concerns an inhalation mask for an inhalation system, an inhalation assembly and a usage of an inhalation mask.

Inhalation masks are commonly used for connecting inhalation systems like chambers, spacers, MDIs or inhalers such as jet or mesh inhalers to faces of users. The inhalation mask should provide a connection from the inhalation system to the user's respiration system through which an aerosol can flow. Leakage between the inhalation mask and the face should be minimized.

Medical guidelines recommend the usage of inhalation masks especially for children's inhalation. Particularly people with difficulties in coordinating their breathing benefit from using an inhalation mask. With many inhalation masks, a user can inhale an aerosol from an inhalation system through the mouth as well as through the nose.

The interface to the user's face is often designed of a flexible material formed as a lip. The combination of this easily deformable structure with a soft material allows a flexible adaption of the inhalation mask to the face geometry und thus a high tightness.

Currently many inhalation masks are used in an inefficient way. They are pressed to hard or soft onto faces resulting in an insufficient sealing. Examples of such previously known inhalation masks are, among others, derivable from EP 3 082 924 A1, WO 2011/121463 A1, WO 2013/186650 A1, WO 2018/104253 A1, WO 2017/158471 A1, WO 2020/073840 A1, US 2010/294281 A1, US 2017/128689 A1, WO 2021/019312 A1, and EP 3 347 076 A1.

Due to the flexibility and the adapted material characteristics, very high pressing forces can compress the lip so much that with a multicomponent mask a hard component can come into direct contact with the face of a patient reducing wearing comfort and tightness. Reduced tightness can occur with insufficient pressing forces as well.

The present invention is directed towards providing an alternative inhalation mask to improve the situation described above.

This object is achieved by an inhalation mask for an inhalation system according to independent claim 1, and/or an inhalation assembly according to independent claim 9, and/or a usage of an inhalation mask according to independent claim 10. Distinct embodiments are derivable from the dependent claims. The inhalation mask for the inhalation system comprises a mask body with a port that can be connected to the inhalation system, and an opening with an elastic sealing lip being equipped for joining a face in such a way that the nose, the mouth or both of them are surrounded by the sealing lip. The sealing lip comprises a stowing area and a remaining area arranged adjacent to the stowing area. The stowing area cannot be seen by a user from an outside of the mask or has lost contact to an ambient environment outside the mask, when the mask is pressed onto the face with an adequate force, and the remaining area remains visible or is still in contact with the ambient environment, when the mask is pressed onto the face with the adequate force. In a sealing position, the stowing area shows at least in one section one or more of the conditions laying on the face, being compressed or bent to an inner side of the inhalation mask. The inhalation mask is provided with an identification distinguishing the stowing area from the remaining area and being configured to show if the adequate force is applied.

By pressing the inhalation mask onto the face, particularly onto the face of a child, the lip is deformed and may be compressed. Therefore, the visible part of the lip is reduced. The distance being required for the deformation of the lip correlates directly with the force being applied and with the tightness. This is due to the geometry of the lip and the material characteristics. The identification shows if an adequate pressure is applied. Therefore, this inhalation mask provides a tool for the user or a caregiver that helps to realize an efficient connection between a face and the mask.

Visualization of a good mask position helps preventing, that caregivers, especially parents press the inhalation mask onto the face, especially a child's face with too high forces, as this leads to a reduced wearing comfort as well as leakage. Thus, adherence and therapy success can be improved. Besides caregivers can get a sense of security regarding correct positioning of the mask.

Improved wearing comfort can lead to a higher adherence. Caregivers can find out the intense or the force needed to press the mask onto the face for achieving a tight position having a high wearing comfort for the user or child and at the same time satisfies the necessary tightness for a high effectiveness of the medical therapy.

Inhalation masks are particularly used in the treatment of respiratory conditions or diseases affecting humans as adults, children, toddlers, infants or newborns. They can be used for the treatments of animals as well.

An inhalation mask includes a mask body shaped to receive a user's nose, mouth or both of them. An exterior surface of the mask body is exposed to an ambient environment.

Inhalation masks are available in different specifications: For example, with and without valves, holes and other openings. The interface to the user's face is often designed of a flexible material formed as a lip. They are used for different inhalation systems as jet nebulizers, membrane nebulizers, spacers, chambers, and others.

Inhalation masks differ in their materials and structure. Common structures are one material structures or multicomponent structures. Polyvinyl chloride (PVC), silicone, polycarbonate (PC), polypropylene (PP), thermoplastic elastomer (TPE) and other materials are used in inhalation masks.

The inhalation system preferably comprises an aerosol generation device. An aerosol generating device preferably comprises a nebulizer, a sprayer, a humidifier, a compressed air nebulizer, an air atomizer, an electronic nebulizer, an ultrasonic nebulizer, an electrohydrodynamic nebulizer, an electrostatic nebulizer, a membrane nebulizer, a nebulizer with a vibrating membrane, an electronic nebulizer with a vibrating membrane, a mesh nebulizer, a jet nebulizer, an MDI, an inhaler, a powder atomizer, a dry powder inhaler (DPI) or a combination thereof. The MDI comprises a pressurized canister with a medicament and a propellant.

The inhalation system may comprise a device for providing aerosols. The device for providing aerosols preferably comprises a spacer or a chamber. Devices for providing aerosols are preferably devices being arranged for use with MDI's. Devices for providing aerosols comprise spacers or chambers, arranged to receive aerosol preferably supplied by MDI's, so that users can inhale the aerosol.

Spacers do not have any valves. Chambers or holding chambers have inspiratory valves and preferably also expiratory valves.

Aerosols for therapeutic purposes are generated and delivered to a desired location within a user or patient's body with aerosol delivery devices. Aerosols are mixtures of solid or liquid particulates and a gas. Aerosols are preferably intended for application onto or into parts of the human or animal body, such as skin, body cavities, body openings, nose, paranasal sinuses, maxillary sinuses, frontal sinuses, sphenoidal sinuses, ethmoid sinuses, pharynx, larynx, trachea, lung, main bronchus, bronchi, bronchioles, alveoli, joints or abdominal cavity.

Aerosols may be employed, to prevent, diagnose or treat diseases of humans and animals, or to immunize against diseases. They are useful for the treatment of respiratory conditions or diseases affecting adults, children, toddlers, infants or newborns.

Preferably, the sealing lip comprises a soft component like an elastic material that returns to its original size and shape when an influence or force is removed. The elastic material may comprise silicone or an elastomer, especially a thermoplastic elastomer.

The soft component may comprise a transparent material and a nontransparent material. One of these materials may be arranged in the stowing area and the other material may be arranged in the remaining area.

The sealing lip comprises a stowing area, being deformed when the mask is pressed on a face correctly. The stowing area is equipped for being deformed such that it lays on the face, is bent to an inside of the mask body or is compressed when being pressed on a face correctly. Some or all of these may occur together. This deformation effects that the stowing area of the sealing lip becomes invisible or is at least harder to view from an outside of the mask. The mask may be formed at least partly by a transparent material. In one embodiment the stowing area is formed by a transparent material. When the mask is deformed in a correct way, the transparent material is not viewable. When laying on the face or being bent to an inside of the mask body, the stowing area may still be viewable through this material but is at least harder to view.

The remaining area is a part of the sealing lip that is arranged adjacent to the stowing area and has contact to the outside environment of the mask when it is used correctly.

The identification allows distinguishing the stowing area from the remaining area. This can be realized in multiple ways. Preferably, the stowing area and the remaining area are optically designed in a different way. In one embodiment, they are designed to feel differently. In one embodiment, a separation of stowing area and remaining area can be seen or felt. The separation may be a rib, notch, groove or line.

The two areas may show different roughnesses, or a glossy finish may be applied to one of the areas. Also, a coloring may be provided in one of the areas. The coloring can be provided only in some areas of the identification, so that for example stripes or dots appear in the identification area. Also, it is possible to provide different surface structures or textures. The stowing area or the remaining area may be transparent.

Further embodiments of the identification comprise one more of the following identification elements: grooves, recesses, bulges, bubbles and embossments. The recesses, bulges, bubbles and embossments may be circular or oval. The identification elements may be provided in the soft component. They visualize the required deformation of the mask. Identification elements in different dimensions, which are arranged by size and distance to a boundary between a remaining area and a stowing area can provide the user with a better information regarding actual status and the status of a good pressure for sealing. The identification may comprise rows of circular bulges. The bulges may be arranged parallel to a boundary between a remaining area and a stowing area. The bulges that are in the closest row to the boundary may have the biggest diameter. The bulges in the row being furthest away from the boundary may show the smallest diameter and the bulges in the row being between these two rows exhibit a diameter with dimensions that are between the dimensions of the bulges in the closest row and the furthest row. This way the user sees the different dimensions of the bulges that are being stowed away and thus disappearing with growing pressure. Therefore, it may be easier for the user to assess how much more pressure he should apply when he is mounting the mask.

Certainly, it is possible to combine several options.

In one embodiment the identification comprises two different materials.

In one embodiment the identification comprises a material out of the following materials silicone and thermoplastic elastomer for the stowing area and another material out of polyvinyl chloride, polycarbonate and polypropylene for the remaining area. In one embodiment two materials with different surface feels are used for the identification.

According to an embodiment the identification may comprise a first color for the stowing area and a different color for the remaining area.

The identification may comprise a marking or a surface structure.

The marking or surface structure may be provided in the stowing area or the remaining area. In one embodiment, the marking or surface structure is provided in the stowing area and the remaining area. It may be appropriate to provide different configurations of the marking or surface structure in different areas. In one embodiment, the marking or surface structure is arranged as a pattern. The marking or surface structure may comprise different material thicknesses, different surface roughness's, a matt area, a shiny area, a polished area, a bubble, a bulge, a protuberance, a recess, a color, a finish, an imprint or a scratch.

In an embodiment the marking or surface structure comprises several elements.

The several elements or items may be dots, polygons, ellipses, circles, bulges or recesses.

The marking or surface structure may at least to some extend be provided in the remaining area, wherein the dimensions of the elements are related to their distance to the stowing area.

The dimensions of elements arranged closer to the stowing area may be bigger than the dimensions of elements arranged further away from the stowing area. This can give an indication regarding the pressing force left to a good sealing.

The surface structure may comprise a rib, notch or groove.

The rib may be arranged between the stowing area and the remaining area. In one embodiment several ribs are provided on the remaining area.

In an embodiment the identification is invisible from the outside of the mask body when the mask is pressed on the face in a correct way.

The identification may be a color or marking arranged in the stowing area. When the mask is pressed on the face in a correct way, the stowing area with the identification should be invisible or at least harder to view, as it lays on the face, is compressed or bent to the inside of the mask body.

One aspect of the invention is an inhalation assembly comprising an inhalation mask and an inhalation system. The inhalation system may be a system being arranged for providing a medicament, a gas or an aerosol. Preferably the inhalation system comprises a metered-dose inhaler, a chamber, a spacer, a nebulizer, an atomizer, a jet nebulizer or a vibrating-mesh nebulizer.

It is an aspect of the invention to provide a method for using an inhalation mask comprising the steps of pressing the inhalation mask on a face in such a way that the nose, the mouth or both of them are surrounded by the sealing lip and adjusting the pressure by watching the identification such that the stowing area is deformed that it is invisible from an outside of the mask or loses contact to an ambient environment outside the mask and the remaining area is still viewable or in contact with the ambient environment.

By watching the identification, a caregiver can increase the pressure when he sees that the stowing area is still visible. On the other hand, he can reduce the pressure when he sees that the remaining area is not visible in full. When the remaining area is in contact with the ambient environment, it is possible to see it.

In one embodiment, the identification comprises a deformation indicator or tightness indicator preferably located on a flexible lip marking the correct position visualizing the deformation of the lip for caregivers or parents. The inhalation mask may be equipped with a soft, deformable component for visualization of the deformation path. The required position is marked on the lip.

The tightness indicator can support to visualize the required deformation path for achieving a high tightness by marks on the lip. This can be achieved by a circumferential marking on the lip, disappearing by compressing the lip, so that caregivers or parents can apply the mask with the correct force.

Embodiments of the identification comprise color marks, symbols, naps, recesses, grooves or lines. Recesses or naps may be realized as bubbles. Furthermore, it is suggested to use geometrically forms, which can be pushed together for constituting a defined graphic or contour in the correct position. Surface textures as for example eroded structures can be used as identification as well.

In one embodiment the identification comprises a marking or surface structure, which shows a symbol when the inhalation mask is pressed on the face correctly. This can be realized by structures or markings, which are distanced from each other when the seal is unstressed. When stress is applied to the seal, the structures or markings can move closer together, so that a symbol is created.

### Brief Description of the Drawings

Fig. 1 shows an inhalation mask with an identification;
Fig. 1a shows a view of an identification being formed like waves;
Fig.1b shows a view of an identification being formed like bubbles;
Fig.1c shows a view of an identification being colored;
Fig.1d shows a view of an identification being formed as a line, rib, groove or notch;
Fig. 2 shows an inhalation mask with an identification being arranged in a distance from the sealing border;
Fig. 2a shows a view of an identification being formed like waves;
Fig. 2b shows a view of an identification being formed like bubbles;
Fig. 2c shows a view of an identification being colored;
Fig. 2d shows a view of an identification being formed as several lines or small ribs;
Fig. 3 shows an inhalation mask with an identification being arranged on the remaining area;
Fig. 3a shows a view of an identification being formed like waves;
Fig. 3b shows a view of an identification being formed like bubbles;
Fig. 3c shows a view of an identification being colored;
Fig. 4 shows an inhalation mask with an identification;
Fig. 4a shows a view of an identification being formed like waves;
Fig. 4b shows a view of an identification being formed like bubbles;
Fig. 4c shows a view of an identification being colored;
Fig. 4d shows a view of an identification being formed as a line;
Fig. 5 shows an inhalation mask with an identification;
Fig. 5a shows a view of an identification being formed like waves;
Fig. 5b shows a view of an identification being formed like bubbles;
Fig. 5c shows a view of an identification being colored;
Fig. 6 shows a sideview of an inhalation mask;
Fig. 7 shows a view of an inhalation mask being applied to a face;
Fig. 8 shows a sideview of an inhalation mask with a sealing lip being adapted to the contour of a face;
Fig. 9 shows the inhalation mask of figure 8 being applied to a face;
Fig. 10 shows a sideview of an inhalation mask with a sealing lip being adapted to the contour of a face;
Fig. 11 shows the inhalation mask of figure 10 being applied to a face;
Fig. 12 shows an inhalation assembly with a chamber;
Fig. 13 shows an inhalation assembly; and
Fig. 14 shows an inhalation assembly with a nebulizer.

### Detailed description of the preferred embodiments of the invention

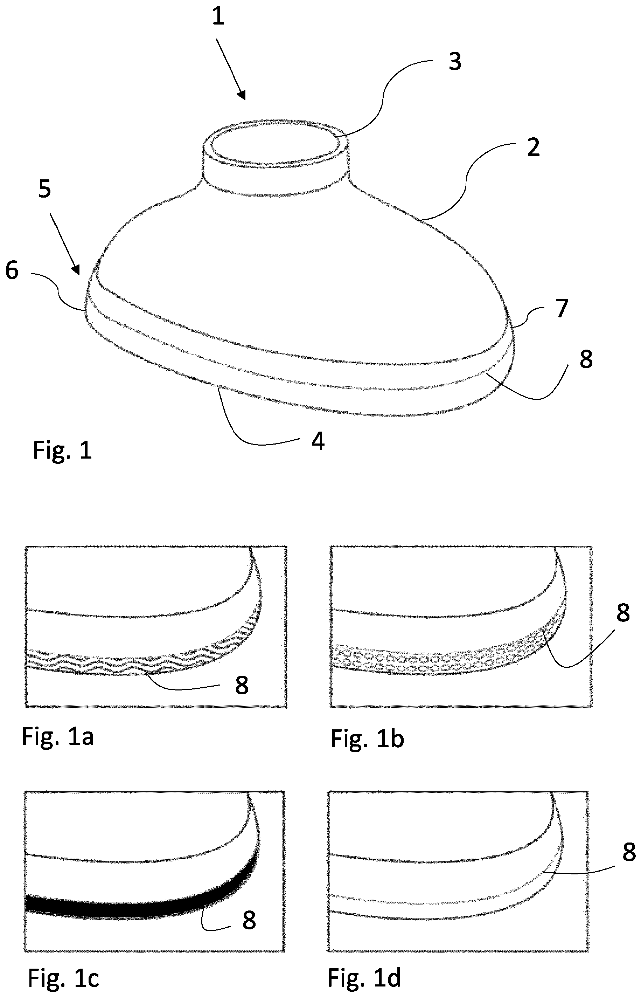

Fig. 1 shows an inhalation mask 1 with a mask body 2, a port 3 that can be connected to an inhalation system, an opening 4 and a circumferential sealing lip 5 being arranged at the opening 4. The circumferential sealing lip 5 is intended for applying the mask to the face of a user. It shows a stowing area 6 and a remaining area 7.

When the mask 1 is pressed onto a face with an adequate force, the stowing area 6 cannot be seen by the user anymore. It lays on the face or is compressed so that it is not visible from the outside. The remaining area 7 remains visible in this condition.

The line between the stowing area 6 and the remaining area 7 is an identification 8 that allows the user to distinguish the stowing area 6 from the remaining area 7. This way the user can see whether the pressing force is adequate or if he must increase or reduce the pressing force. The user can optimize the pressing force to generate a good sealing between the mask 1 and the face.

Figure 1a shows an embodiment of the identification 8 shown in figure 1. Here the identification 8 is realized by a surface structure that is formed on the stowing area 6. In this case the surface structure shows the form of waves.

Figure 1b shows an embodiment of the identification 8 shown in figure 1. The identification 8 is realized by a surface structure that is formed on the stowing area 6 as well. In this embodiment the surface structure shows the form of bubbles.

Figure 1c shows an identification 8 that is realized by a difference in colors between the stowing area 6 and the remaining area 7.

Figure 1d shows an identification 8 that is formed by a line, rib, groove or notch being arranged between the stowing area 6 and the remaining area 7.

There are more options available for allowing the user to distinguish between a stowing area 6 and a remaining area 7, like for example different roughnesses of the areas or a glossy finish. The coloring can be provided only in some areas of the identification 8, so that for example stripes or dots appear in the identification 8 area. Also, it is possible to provide different surface structures or textures. A stowing area 6 or a remaining area 7 may be transparent. Certainly, it is possible to combine several options.

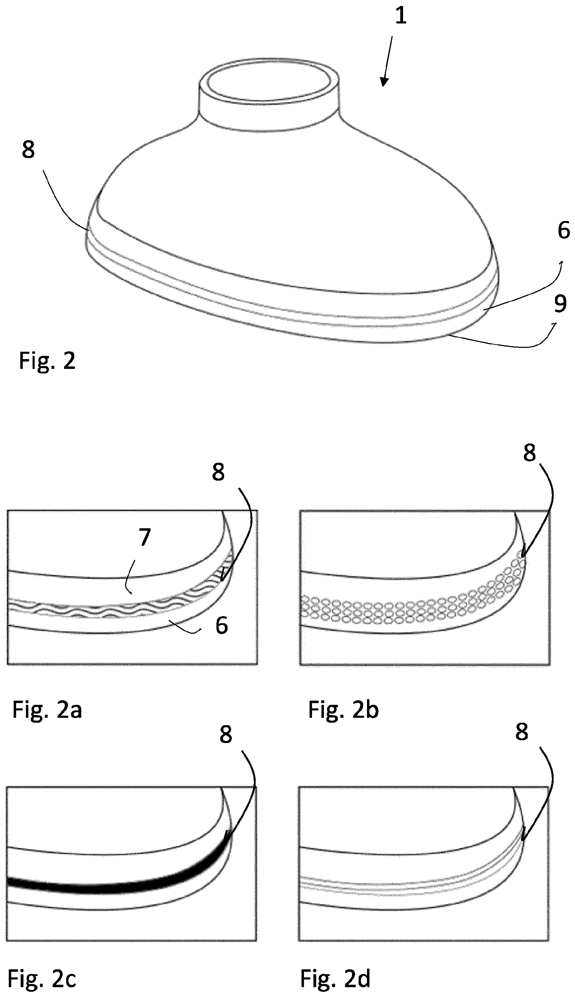

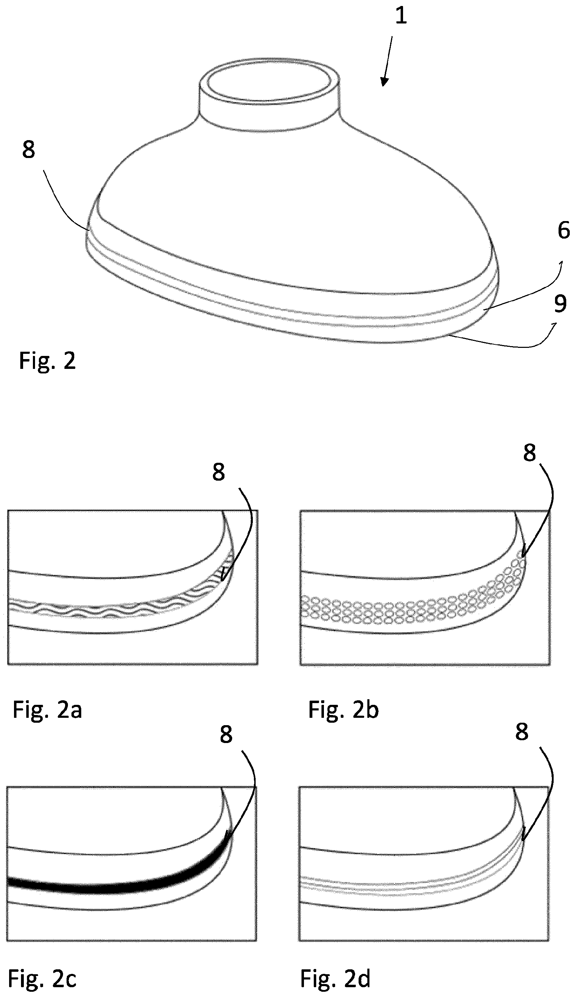

Fig. 2 shows an inhalation mask 1 like the mask shown in figure 1 with an identification 8 being arranged on the stowing area 6 in a distance from the sealing border 9.

When the mask 1 is pressed onto a face with an adequate force the identification 8 cannot be seen from the user anymore. It lays on the face or is compressed so that it is not visible from the outside. When the user lays the mask 1 onto the face and starts increasing pressure, at first the stowing area 6 without the identification 8 will disappear. After that with still increasing pressure the area with identification 8 will disappear. Thus, the user sees that he is close to the optimized pressure when the area with the identification 8 starts disappearing. The user knows that the pressure increase needed is only very small. Therefore, he can act more carefully from this point avoiding uncomfortable pressure for the patient.

Figure 2a shows an embodiment of the identification 8 shown in figure 2. Here, the identification 8 is realized by a surface structure that is formed on the section of the stowing area 6 that is adjoining the remaining area 7. In this case the surface structure shows the form of waves.

Figure 2b shows an embodiment of the identification 8 like the identification 8 shown in figure 2a. Here, the identification 8 is realized by a surface structure showing bubbles. The bubbles cover a broad area around the contact area of the stowing area and the remaining area. This way a transition area for an adequate pressure is marked. It may be easier for user to adjust the pressure according to the transition area and avoid stress by trying to apply an ideal pressure.

Figure 2c shows an embodiment of the identification 8 like the identification 8 shown in figures 2a and 2b. Here, the identification 8 is realized by a different color of the identification.

Figure 2d shows an identification 8 arranged in the same area as the identification 8 shown in figures 2a, 2b and 2c. Here the identification 8 is realized by several lines or small ribs.

It is possible to provide an identification 8 like the one shown in figure 2 in a slightly different area. The identification 8 may be arranged in the remaining area 7 close to the stowing area 6. This way only a section of the remaining area 7 is provided with a special structure or color.

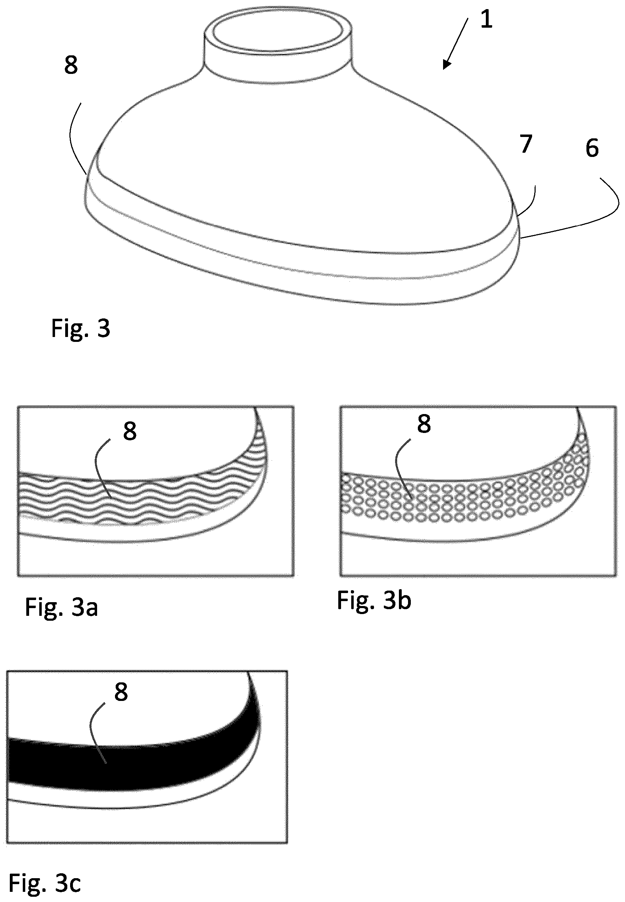

Fig. 3 shows an inhalation mask 1 like the masks shown in figure 1 and figure 2 with an identification 8 being arranged on the remaining area 7.

When the mask 1 is pressed onto a face with an adequate force the identification 8 can be seen from the user in full. The unmarked stowing area 6 lays on the face or is compressed so that it is not visible from the outside. When the user lays the mask 1 onto the face and starts increasing pressure, the stowing area 6 without the identification 8 will disappear.

Figures 3a, 3b and 3c show embodiments of the identification 8 like surface structures with waves or bubbles and colors.

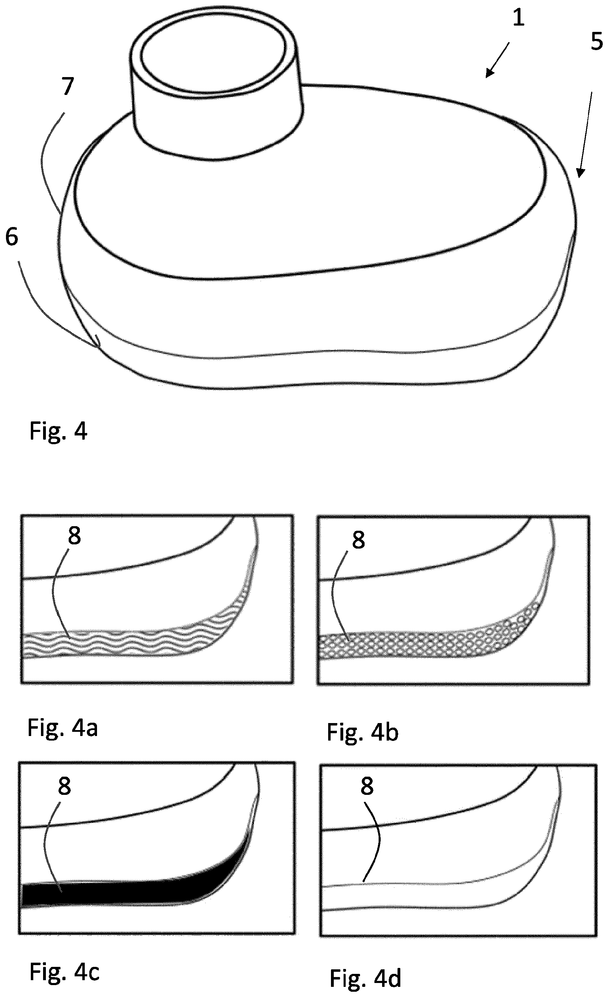

Fig. 4 shows an inhalation mask 1 like the mask shown in figure 1. A difference between the inhalation mask 1 shown in figure 1 and the inhalation mask 1 shown in figure 4 is the configuration of the circumferential sealing lip 5. The sealing lip 5 shown in figure 1 possesses a mainly flat contour and the sealing lip 5 shown in figure 4 shows a contour that is more adapted to a face. It provides room for a nose and the jowls. The sealing lip 5 is thicker around the chin and between the nose and the jowls and thinner around the nose and the jowls. Therefore, it provides better sealing capabilities than the mask 1 shown in figure 1. The stowing area 6 has the same thickness in all areas. The remaining area 7 is thinner around the nose and the jowls than in the other areas. It is possible to allocate the sealing material in other ways to the stowing area 6 and the remaining area 7. For example, the thickness of the remaining area 7 may be of the same thickness in all sections and the thickness of the stowing area 6 may be adapted to the face contour. Also, it is possible to adapt both areas to the face contour.

Figures 4a, 4b, 4c, and 4d show different embodiments of the identification 8 as figures 1a, 1b, 1c and 1d. As shown in the description of figures 1, 1a, 1b, 1c and 1d many options for marking the identification 8 are possible. The surface structures waves and bubbles, the coloring and the line or rib are just examples.

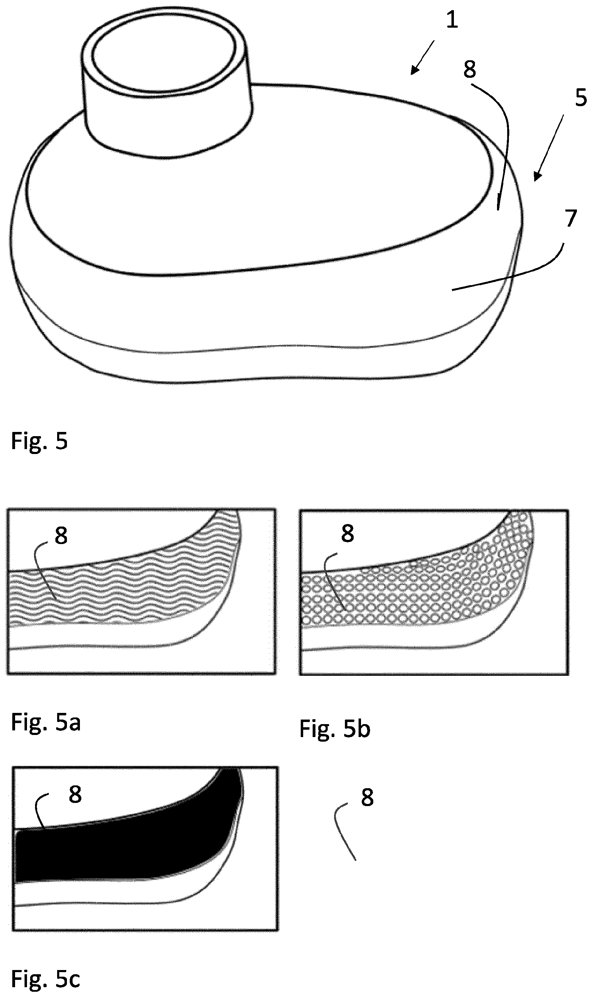

Fig. 5 shows an inhalation mask 1 like the mask shown in figure 3. A difference between the inhalation mask 1 shown in figure 3 and the inhalation mask 1 shown in figure 5 is the configuration of the circumferential sealing lip 5. The sealing lip 5 is formed like the sealing lip 5 shown in Figure **4****.** The identification 8 is arranged on the remaining area 7 like in mask 1 shown in figure 3.

Figures 5a, 5b and 5c show different embodiments of the identification 8 that can be used with the mask 1 shown in figure 5. These embodiments are equivalent to the embodiments described with figures 3a, 3b and 3c. These embodiments are examples. As described above many embodiments for the identification 8 are possible.

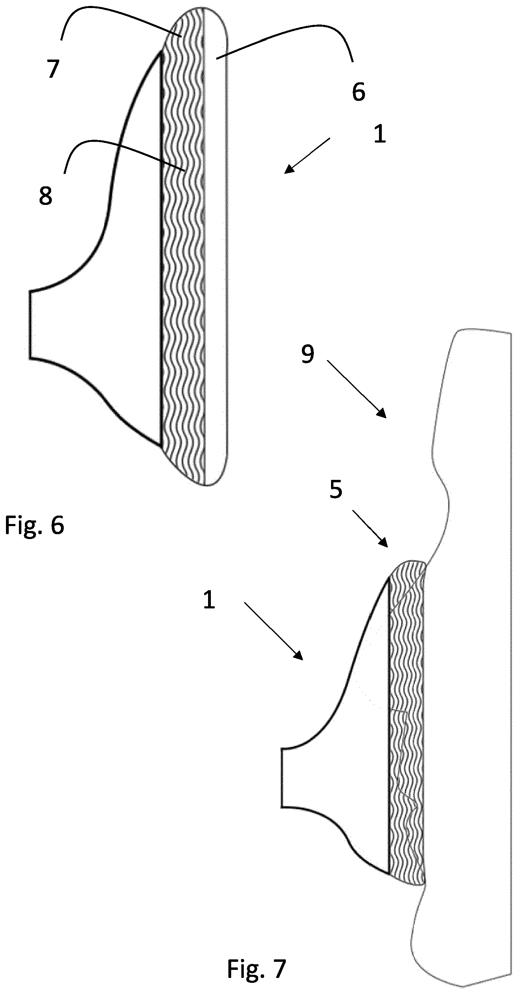

Figure 6 shows a sideview of a mask 1 like mask 1 shown in figure 3 with an embodiment of the identification 8 as shown in figure 3a. The identification 8 marks the remaining area 7. Adjacent to the identification 8 is the stowing area 6. The identification 8 is a marking for visualization of the deformation trajectory or deformation path. It shows to what extend the inhalation mask 1 has to be deformed in order to provide a good sealing.

Figure 7 shows the mask 1 shown in figure 6 being pressed on a face 9 with an optimized pressure force. The mask is deformed and the stowing area 6 is not viewable. It lays on the face 9 or is compressed. In this condition the mask 1 shows a good sealing condition and does not hurt the patient. The user pressing the mask 1 on the face 9 gets feedback regarding the pressuring force. He sees how much material of the sealing lip 5 should disappear and can adjust the pressure accordingly.

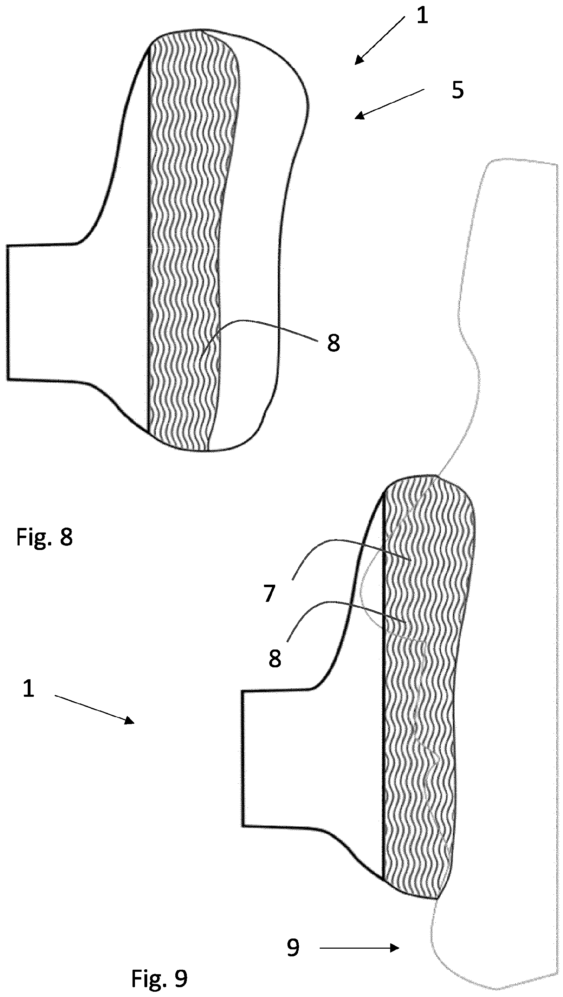

Figure 8 shows a side view of a mask 1 like mask 1 shown in figure 6. The difference between the two masks 1 is that figure 8 shows a sealing lip 5 being adapted to the contour of the face. Masks with adapted contour are described with figures 4 and 5. The embodiment of the identification 8 is described with figure 5a.

Figure 9 shows the mask 1 shown in figure 8 being pressed onto a face 9 with an optimized pressure force. The difference to the situation shown in figure 7 is that the contour of the remaining area 7 is adapted to the face 9 in a better way.

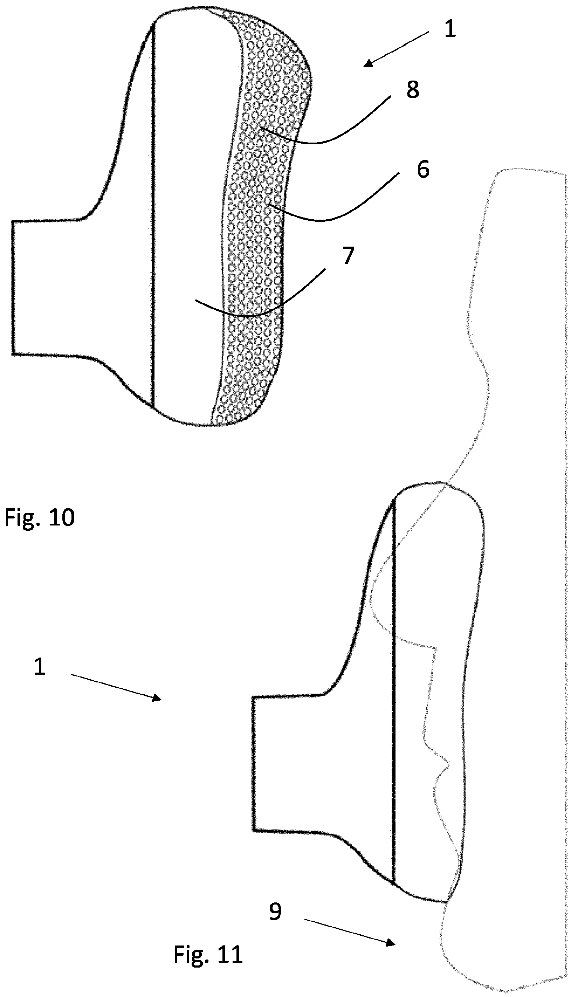

Figure 10 shows a side view of a mask 1 like mask 1 shown in figure 8. A difference to the mask 1 shown in figure 8 is the identification 8 being arranged in the stowing area 6 instead of the remaining area 7. Another difference is that the identification 8 is realized by bubbles instead of waves.

Figure 11 shows the mask 1 shown in figure 10 being pressed onto a face 9 with an optimized pressure force. The identification 8 on the stowing area 6 is not viewable from the outside anymore.

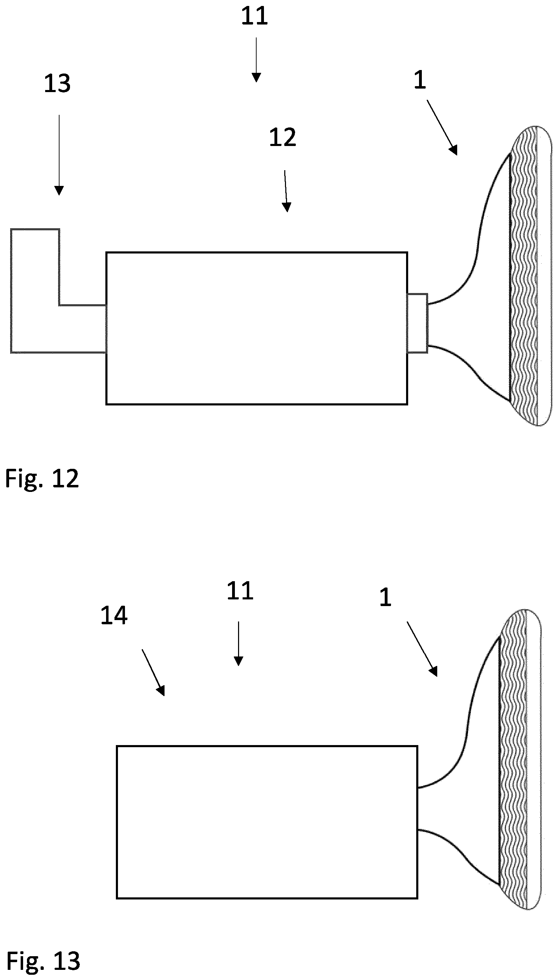

Fig. 12 shows an inhalation assembly 11 comprising a mask 1, a chamber 12 and a metered-dose inhaler (MDI) 13. Upon activation the MDI 13 releases an aerosol into the chamber 12. Chamber 12 is attached to the mask 1 which can be applied to a patient's face, so that the patient can inhale the aerosol.

Fig. 13 shows an inhalation assembly 11 comprising a mask 1 and an inhalation system 14. The inhalation system 14 may comprise a nebulizer like a jet nebulizer, a vibrating membrane nebulizer or any other inhalation system 14. Inhalation system 14 is attached to the mask 1 which can be applied to a patient's face, so that the patient can inhale a medicament, gas, aerosol or whatever the inhalation system 14 provides.

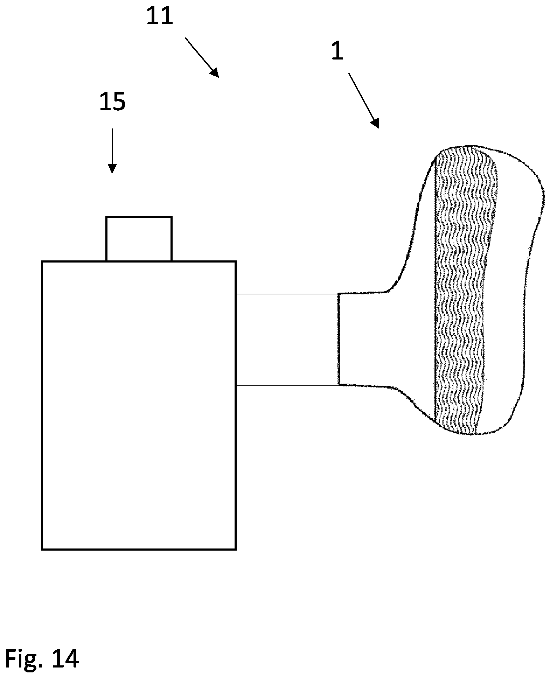

Fig. 14 shows an inhalation assembly 11 comprising a mask 1 and a nebulizer 15. In this example nebulizer 15 is a jet nebulizer.

### List of Reference Signs

- 1: mask
- 2: mask body
- 3: port
- 4: opening
- 5: sealing lip
- 6: stowing area
- 7: remaining area
- 8: identification
- 9: face
- 11: inhalation assembly
- 12: chamber
- 13: MDI
- 14: inhalation system
- 15: nebulizer

## Claims

1. Inhalation mask (1) for an inhalation system (14) comprising
a mask body (2) with
a port (3) that can be connected to the inhalation system (14), and
an opening (4) with an elastic sealing lip (5) being equipped for joining a face (9) in such a way that the nose, the mouth or both of them are surrounded by the sealing lip (5),
and **characterized by**
the sealing lip (5) comprising a stowing area (6), which cannot be seen by a user from an outside of the mask or which has lost contact to an ambient environment outside the mask, when the mask (1) is pressed onto the face (9) with an adequate force, and a remaining area (7) arranged adjacent to the stowing area (6), which remains visible or which is still in contact with the ambient environment, when the mask (1) is pressed onto the face (9) with the adequate force,
wherein,
in a sealing position, the stowing area (6) shows at least in one section one or more of the conditions:
laying on the face, being compressed, or being bent to an inner side of the inhalation mask (1), and wherein
the inhalation mask (1) is provided with an identification (8) distinguishing the stowing area (6) from the remaining area (7) and being configured to show if the adequate force is applied.

2. Inhalation mask (1) according to claim 1, **characterized in that** the identification (8) comprises two different materials.

3. Inhalation mask (1) according to any of the preceding claims, **characterized in that** the identification (8) comprises a first color for the stowing area (6) and a different color for the remaining area (7).

4. Inhalation mask (1) according to any of the preceding claims, **characterized in that** the identification (8) comprises a marking or a surface structure.

5. Inhalation mask (1) according to claim 4, **characterized in that** the marking or surface structure comprises several elements.

6. Inhalation mask (1) according to claim 5, **characterized in that** the marking or surface structure is at least to some extend provided in the remaining area (7), wherein the dimensions of the elements are related to their distance to the stowing area (6).

7. Inhalation mask (1) according to claim 4, 5 or 6, **characterized in that** the surface structure comprises a rib, notch or groove.

8. Inhalation mask (1) according to claim 1 or 2, **characterized in that** the identification (8) is invisible from the outside of the mask body (2) when the mask (1) is pressed on the face (9) in a correct way.

9. Inhalation assembly (11) comprising an inhalation mask (1) according to one of the preceding claims and an inhalation system (14).

10. Method for using an inhalation mask (1) according to any of claims 1 to claim 8,
comprising the steps of
pressing the inhalation mask (1) on a face (9) in such a way that the nose, the mouth or both of them are surrounded by the sealing lip (5) and
adjusting the pressure by watching the identification (8) such that the stowing area (6) is deformed such that it is invisible from an outside of the inhalation mask (1) or loses contact to an ambient environment outside the mask and the remaining area (7) is still viewable or in contact with the ambient environment.

## Patentansprüche

1. Inhalationsmaske (1) für ein Inhalationssystem (14), umfassend ein Maskenkörper (2) mit einer Öffnung (3), die mit dem Inhalationssystem (14) verbunden werden kann, und eine Öffnung (4) mit einer elastischen Dichtlippe (5), die so ausgebildet ist, dass sie ein Gesichtsteil (9) aufnimmt, sodass die Nase, der Mund oder beide von der Dichtlippe (5) umschlossen sind, und **dadurch gekennzeichnet, dass** die Dichtlippe (5) einen Staubereich (6) umfasst, der für einen Benutzer von außerhalb der Maske nicht sichtbar ist oder der den Kontakt zu einer Umgebung außerhalb der Maske verloren hat, wenn die Maske (1) mit einer ausreichenden Kraft auf das Gesicht (9) gedrückt wird, und einen an den Staubereich (6) angrenzend angeordneten übrigen Raum (7), der sichtbar bleibt oder der noch in Kontakt mit der Umgebung steht, wenn die Maske (1) mit der ausreichenden Kraft auf das Gesicht (9) gedrückt wird, wobei der Staubereich (6) in einer Dichtungsstellung zumindest in einem Abschnitt eine oder mehrere der folgenden Bedingungen aufweist:
auf dem Gesicht liegend, zusammengedrückt oder zu einer inneren Seite der Inhalationsmaske (1) gebogen, und wobei die Inhalationsmaske (1) mit einer Kennzeichnung (8) bereitgestellt ist, die den Staubereich (6) vom übrigen Raum (7) unterscheidet und so ausgestaltet ist, dass sie anzeigt, ob die ausreichende Kraft aufgebracht wird.

2. Inhalationsmaske (1) nach Anspruch 1, **dadurch gekennzeichnet, dass** die Kennzeichnung (8) zwei verschiedene Materialien umfasst.

3. Inhalationsmaske (1) nach einem der vorstehenden Ansprüche, **dadurch gekennzeichnet, dass** die Kennzeichnung (8) eine erste Farbe für den Staubereich (6) und eine andere Farbe für den übrigen Raum (7) umfasst.

4. Inhalationsmaske (1) nach einem der vorstehenden Ansprüche, **dadurch gekennzeichnet, dass** die Kennzeichnung (8) eine Markierung oder eine Oberflächenstruktur umfasst.

5. Inhalationsmaske (1) nach Anspruch 4, **dadurch gekennzeichnet, dass** die Markierung oder Oberflächenstruktur mehrere Elemente umfasst.

6. Inhalationsmaske (1) nach Anspruch 5, **dadurch gekennzeichnet, dass** die Markierung oder Oberflächenstruktur zumindest in teilweise in dem übrigen Raum (7) bereitgestellt ist, wobei die Abmessungen der Elemente in Beziehung zu ihrem Abstand zum Staubereich (6) stehen.

7. Inhalationsmaske (1) nach Anspruch 4, 5 oder 6, **dadurch gekennzeichnet, dass** die Oberflächenstruktur eine Rippe, eine Kerbe oder eine Nut umfasst.

8. Inhalationsmaske (1) nach Anspruch 1 oder 2, **dadurch gekennzeichnet, dass** die Kennzeichnung (8) von der Außenseite des Maskenkörpers (2) aus nicht sichtbar ist, wenn die Maske (1) korrekt auf das Gesicht (9) gedrückt wird.

9. Inhalationsanordnung (11), umfassend eine Inhalationsmaske (1) nach einem der vorstehenden Ansprüche und ein Inhalationssystem (14).

10. Verfahren zur Verwendung einer Inhalationsmaske (1) nach einem der Ansprüche 1 bis 8, umfassend die Schritte von
Aufdrücken der Inhalationsmaske (1) auf ein Gesicht (9) derart, dass die Nase, der Mund oder beide von der Dichtlippe (5) umschlossen sind, und
Einstellen des Drucks durch Beobachten der Kennzeichnung (8), sodass der Staubereich (6) derart verformt wird, dass er von außerhalb der Inhalationsmaske (1) nicht mehr sichtbar ist oder den Kontakt zur Umgebung außerhalb der Maske verliert und der übrige Raum (7) weiterhin sichtbar ist oder in Kontakt mit der Umgebung steht.

## Revendications

1. Masque d'inhalation (1) pour un système d'inhalation (14) comprenant un corps de masque (2) avec
un orifice (3) qui peut être connecté au système d'inhalation (14), et
une ouverture (4) avec une lèvre d'étanchéité élastique (5) équipée pour joindre un visage (9) de manière que le nez, la bouche ou les deux soient entourés par la lèvre d'étanchéité (5),
et **caractérisé en ce que**
la lèvre d'étanchéité (5) comprend une zone d'arrimage (6) qui ne peut pas être vue par un utilisateur depuis l'extérieur du masque ou qui a perdu le contact avec un environnement ambiant à l'extérieur du masque,
lorsque le masque (1) est pressé sur le visage (9) avec une force adéquate, et une zone restante (7) disposée à proximité de la zone d'arrimage (6), qui reste visible ou qui est toujours en contact avec l'environnement ambiant, lorsque le masque (1) est pressé sur le visage (9) avec une force adéquate,
dans lequel,
dans une position d'étanchéité, la zone d'arrimage (6) présente au moins dans une section une ou plusieurs des conditions suivantes :
posée sur le visage, comprimée, ou pliée vers un côté intérieur du masque d'inhalation (1), et dans lequel
le masque d'inhalation (1) est doté d'une identification (8) distinguant la zone d'arrimage (6) de la zone restante (7) et étant configurée pour montrer si la force adéquate est appliquée.

2. Masque d'inhalation (1) selon la revendication 1, **caractérisé en ce que** l'identification (8) comprend deux matériaux différents.

3. Masque d'inhalation (1) selon l'une quelconque des revendications précédentes, **caractérisé en ce que** l'identification (8) comprend une première couleur pour la zone d'arrimage (6) et une couleur différente pour la zone restante (7).

4. Masque d'inhalation (1) selon l'une quelconque des revendications précédentes, **caractérisé en ce que** l'identification (8) comprend un marquage ou une structure de surface.

5. Masque d'inhalation (1) selon la revendication 4, **caractérisé en ce que** le marquage ou la structure de surface comprend plusieurs éléments.

6. Masque d'inhalation (1) selon la revendication 5, **caractérisé en ce que** le marquage ou la structure de surface est au moins dans une certaine mesure fourni(e) dans la zone restante (7), dans lequel les dimensions des éléments sont liées à leur distance par rapport à la zone d'arrimage (6).

7. Masque d'inhalation (1) selon la revendication 4, 5 ou 6, **caractérisé en ce que** la structure de surface comprend une nervure, encoche ou rainure.

8. Masque d'inhalation (1) selon la revendication 1 ou 2, **caractérisé en ce que** l'identification (8) est invisible depuis l'extérieur du corps de masque (2) lorsque le masque (1) est pressé sur le visage (9) d'une manière correcte.

9. Ensemble d'inhalation (11) comprenant un masque d'inhalation (1) selon l'une des revendications précédentes et un système d'inhalation (14).

10. Procédé pour utiliser un masque d'inhalation (1) selon l'une quelconque des revendications 1 à 8, comprenant les étapes consistant à
presser le masque d'inhalation (1) sur un visage (9) de manière que le nez, la bouche ou les deux soient entourés par la lèvre d'étanchéité (5) et
ajuster la pression en observant l'identification (8) de sorte que la zone d'arrimage (6) est déformée de sorte qu'elle est invisible depuis un extérieur du masque d'inhalation (1) ou perd le contact avec un environnement ambiant à l'extérieur du masque et la zone restante (7) reste visible ou en contact avec l'environnement ambiant.
